# EUROPÄISCHE PATENTSCHRIFT

(11) **EP 3 611 896 B1**
(45) Veröffentlichungstag und Bekanntmachung des Hinweises auf die Patenterteilung: **31.08.2022**
(21) Anmeldenummer: 19000349.1
(22) Anmeldetag: 29.07.2019
(51) Int. Cl.: A61M 16/00

(54) **SICHERE KOMMUNIKATION ZWISCHEN EINEM SERVER UND EINEM BEATMUNGSSYSTEM**
SECURE COMMUNICATION BETWEEN A SERVER AND A BREATHING SYSTEM
COMMUNICATION SÉCURISÉE ENTREE UN SERVEUR ET UN SYSTÈME DE RESPIRATION

(30) Priorität: 13.08.2018 DE 102018006352
(43) Veröffentlichungstag der Anmeldung: 19.02.2020
(73) Patentinhaber: Löwenstein Medical Technology S.A., 2557 Luxembourg (LU)
(72) Erfinder: Schwaibold, Matthias, 76228 Karlsruhe (DE); Bychkov, Igor, 76275 Ettlingen (DE); Kopaigorenko, Maria, 76133 Karlsruhe (DE); Skiba, Alexander, 76297 Stutensee (DE)
(74) Vertreter: Marx, Thomas

(56) Entgegenhaltungen:
- EP-A1- 3 340 095
- WO-A1-2018/151778
- US-B1- 9 215 075
- PULKKIS GORAN ET AL: "Secure and Reliable Internet of Things Systems for Healthcare", 2017 IEEE 5TH INTERNATIONAL CONFERENCE ON FUTURE INTERNET OF THINGS AND CLOUD (FICLOUD), IEEE, 21. August 2017 (2017-08-21), Seiten 169-176, XP033257869, DOI: 10.1109/FICLOUD.2017.50 [gefunden am 2017-11-16]
- ALROMAIHI SARA ET AL: "Cyber Security Challenges of Deploying IoT in Smart Cities for Healthcare Applications", 2018 6TH INTERNATIONAL CONFERENCE ON FUTURE INTERNET OF THINGS AND CLOUD WORKSHOPS (FICLOUDW), IEEE, 6. August 2018 (2018-08-06), Seiten 140-145, XP033418136, DOI: 10.1109/W-FICLOUD.2018.00028 [gefunden am 2018-10-09]

## Beschreibung

Die Erfindung betrifft ein Verfahren zur sicheren Kommunikation in einem Beatmungssystem bei der Ferneinstellung eines Beatmungsgerätes durch einen Server.

Die Erfindung betrifft auch ein Beatmungssystem zur Durchführung des Verfahrens.

Die Erfindung betrifft auch ein Beatmungsgerät zur Durchführung des Verfahrens.

Die EP2392253 offenbart A1 ein Verfahren zur Aktualisierung von Beatmungsgeräten, bei dem ein im Gerät abgespeichertes Betriebsprogramm mindestens teilweise durch ein neues Betriebsprogramm ersetzt wird. Die beschriebene Aktualisierung ist jedoch unspezifisch und nicht gesichert. Die EP 3340095 A1 offenbart ein Verfahren zum Betreiben einer Datenverarbeitungseinrichtung eines Beatmungsgerätes, bei dem für den Zguriff auf Therapiedaten das Hinterlegen eines Berechtigungscodes erforderlich ist. Zudem wird eine Authentifizierung der an der Verbindung beteiligten Geräte beschrieben. Die WO 2018/151778 A1 offenbart die Sicherung und Verschlüsselung von Datenübertragungen eines Beatmungsgerätes sowie die Generierung entsprechender Authentifizierungscodes. Die US 9,215,075 B1 beschreibt ein Verfahren und ein zugehöriges implantierbares Gerät, sowie die sichere und verschlüsselte Übertragung von Therapiedaten, insbesondere über Netzwerke wie dem Internet.

Die Aufgabe der vorliegenden Erfindung ist es, ein Verfahren anzugeben, welches eine einfache und sichere Ferneinstellung des Beatmungsgerätes unterstützt wird.

Die Aufgabe wird gelöst durch ein Verfahren zur sicheren Kommunikation in einem Beatmungssystem zur Ferneinstellung eines Beatmungsgerätes durch einen Server umfassend, dass
- eine Authentifizierung von Beatmungsgerät und Server durchgeführt wird
- eine Kommunikationsverbindung zwischen dem Beatmungsgerät und dem Server gesichert wird
- eine Verschlüsselung der Kommunikation zwischen dem Beatmungsgerät und dem Server vorgenommen wird
- die Integrität der übermittelten Daten/Einstellungen überprüft wird
- eine Kennzeichnung der übermittelten Daten/Einstellungen mit einem Zeitstempel und/oder einer Geräte-ID und/oder einer Firmware-Version stattfindet
- ein Zugriff auf den Server und/oder eine Prüfung der Zugriffsrechte für den Server geschützt wird
- eine eindeutige Zuordnung eines Nutzers zu einem Beatmungsgerät durchgeführt wird

Erfindungsgemäß wird
- eine Vorauswahl geeigneter Einstelloptionen und dazu passender Einstellparameter für ein individuelles Beatmungsgerät durch den Server bereitgestellt, wobei der Server lediglich solche Einstelloptionen und Einstellparameter aus einer Datenbank identifiziert, die zu dem individuellen Beatmungsgerät kompatibel sind und
- die Eignung der vom Server bereitgestellten Einstelloptionen und Einstellparameter durch das Beatmungsgerät geprüft.

Erfindungsgemäß soll die Vertraulichkeit bei der Ferneinstellung eines Beatmungsgerätes geschützt werden. Die Daten sollen nicht abgehört werden können und unbefugten Anwendern nicht sichtbar gemacht werden.

Erfindungsgemäß soll die Integrität (der Daten) bei der Ferneinstellung eines Beatmungsgerätes geschützt werden. Die Daten sollen nicht aus Versehen bei der Kommunikation verfälscht werden können.

Erfindungsgemäß soll ein Manipulationsschutz gewahrt werden. Für nicht-autorisierte Benutzer soll es keine Möglichkeit geben, die Einstellungen des Beatmungsgerätes zu verändern.

Erfindungsgemäß soll die korrekte Funktion des Beatmungsgerätes gewahrt werden. Die gewählten neuen Einstellungen müssen für das Gerät geeignet und von ihm ausführbar sein.

Erfindungsgemäß soll die Transparenz bei der Ferneinstellung gewahrt werden: Die Benutzer von Gerät und Server sollten darüber informiert werden, dass eine Veränderung der Einstellungen stattgefunden hat und ob sie erfolgreich durchgeführt wurde.

Bei dem Aufbau einer Kommunikationsverbindung zwischen einem Server und dem Beatmungsgerät erfolgt eine Authentifizierung von Gerät und Server.

Zur Authentifizierung und Verschlüsselung können generell auch Zertifikate (private / public) verwendet werden, die beispielsweise in dem Beatmungsgerät gespeichert sind (weil sie bereits bei der Herstellung oder nachträglich in einem Speicher des Gerätes abgelegt wurden) und die während der Kommunikation zwischen Server und Gerät über einen separaten Kommunikationskanal mit einer Datenbank auf ihre Gültigkeit hin überprüft werden.

Sobald eine Kommunikationsverbindung aufgebaut ist, erfolgt eine Sicherung der Kommunikationsverbindung.

Erfindungsgemäß ist auch vorgesehen, dass die Kommunikation über die Kommunikationsverbindung verschlüsselt erfolgt.

Bei der Kommunikation erfolgt auch eine Überprüfung der Integrität der Daten. Die Daten sollen nicht (aus Versehen) bei der Kommunikation verfälscht werden können, beispielsweise durch schlechte Datenqualität.

Bei der Kommunikation erfolgt auch eine Kennzeichnung mit einem Zeitstempel und/oder einer Geräte-ID und Firmware-Version. Eine Kennzeichnung kann beispielsweise dadurch erfolgen, dass eine Ergänzung mindestens eines Zeitstempels (beispielsweise für Aktivierung und Empfang) erfolgt, um auch bei asynchroner Datenverarbeitung die chronologische Reihenfolge der angewandten Einstellungen ermitteln und die neueste bekannte Einstellung identifizieren zu können.

Erfindungsgemäß ist auch ein Zugriffsschutz für den Server und eine Prüfung der Zugriffsrechte für jeden Betreuer vorgesehen. Erfindungsgemäß ist auch eine eindeutige Zuordnung eines Nutzers zu einem Beatmungsgerät vorgesehen.

Erfindungsgemäß ist auch vorgesehen, dass der Server für das spezifische Beatmungsgerät, aus einer Vielzahl an Einstelloptionen und Einstellparameter, eine spezifische Vorauswahl geeigneter Einstelloptionen und dazu passender Einstellparameter anbietet. Dies erleichtert die Vorgabe der Einstelloptionen und Einstellparameter und verhindert die Vorgabe unsinniger Kombinationen. Die Vorauswahl geeigneter Einstelloptionen und dazu passender Einstellparameter wird dadurch erreicht, dass der Server das Beatmungsgerät anhand seiner Geräte-ID und/oder des Geräte-Typs und/oder der Seriennummer und/oder der Firmware-Version identifiziert.

Erfindungsgemäß kann eine weitere Prüfung der Eignung der vom Server bereitgestellten Einstellungen durch das Beatmungsgerät erfolgen.

Erfindungsgemäß kann eine weitere Prüfung der vom Server bereitgestellten Einstellungen durch den Nutzer erfolgen. Diese Prüfung ist als zusätzliche Sicherheitsmaßnahme vorgesehen, und sieht vor, dass der Nutzer die Übernahme der Einstellungen am Beatmungsgerät oder für das Beatmungsgerät bestätigt. Erfindungsgemäß kann eine Prüfung der Eignung der vom Server bereitgestellten Einstellungen dadurch erfolgen, dass die Identität des Nutzers verifiziert werden muss.

Erfindungsgemäß kann eine Bestätigung/Quittierung der vom Server bereitgestellten Einstellungen durch den Nutzer erfolgen. Erfindungsgemäß kann eine Deaktivierungsmöglichkeit des Abrufens von Ferneinstellungen vorgesehen sein. Dadurch kann zumindest bei Deaktivierung ein zusätzlicher Schutz vor Ferneinstellung des Beatmungsgerätes erfolgen.

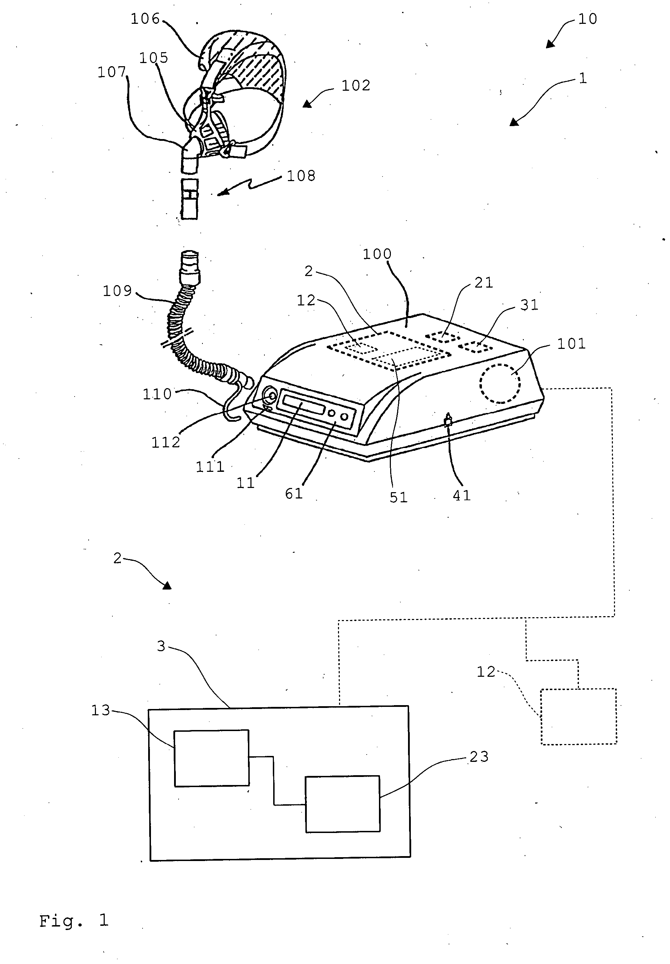

Die Figur 1 zeigt eine stark schematische Darstellung eines erfindungsgemäßen Beatmungssystems.

In der Figur 1 ist ein erfindungsgemäßes Beatmungssystem 10 gezeigt, welches hier ein als Heimbeatmungsgerät 11 bzw. Schlaftherapiegerät eingesetztes Beatmungsgerät 1 umfasst. Das Beatmungsgerät 1 kann aber auch als klinisches Beatmungsgerät, CPAP oder APAP, Hustentherapiegerät oder High-Flow-Gerät 1 ausgebildet sein. Das Beatmungsgerät kann auch mindestens einen Modus zur (nasalen) Highflow-Therapie oder High-Flow Sauerstofftherapie besitzen, der durch die Ferneinstellung ausgewählt und parametriert werden kann. Das Beatmungsgerät 1 ist zur Durchführung des erfindungsgemäßen Verfahrens geeignet und ausgebildet.

Das Beatmungsgerät 1 umfasst eine Beatmungseinrichtung 100, die beispielsweise als Gebläseeinrichtung und/oder Ventileinrichtung 101 ausgeführt ist, zur Erzeugung eines Luftstromes für die Beatmung. Zur Steuerung der Beatmungseinrichtung 100 und zur Erfassung von Therapiedaten ist hier eine Überwachungseinrichtung 21 vorgesehen. Die Bedienung und Einstellung des Beatmungsgerätes 1 erfolgt über eine Nutzerschnittstelle 61 mit Bedienelementen 103 und einer Anzeigeeinrichtung 11.

Das Beatmungsgerät 1 weist eine Atemschnittstelle 102 auf, um den Luftstrom einem Nutzer zur Beatmung zuzuführen. Die hier gezeigte Atemschnittstelle 102 ist eine als Nasalmaske ausgebildete Atemmaske 105. Zur Fixierung der Atemmaske 105 ist eine Kopfhaube 106 vorgesehen. Die Atemschnittstelle 102 kann beispielsweise auch als eine Vollgesichtsmaske, als ein Nasal-Pillow, als ein Tubus oder als eine Larynxmaske ausgestaltet sein.

Zur Verbindung der Atemschnittstelle 102 mit der Beatmungseinrichtung 100 ist ein Verbindungsschlauch 109 vorgesehen, der mittels einer Koppeleinrichtung 112 mit der Beatmungseinrichtung 100 verbunden wird. Über ein Kuppelungselement 107 wird der Verbindungsschlauch 109 mit der Atemschnittstelle 102 verbunden. Zwischen dem Verbindungsschlauch 109 und dem Kuppelungselement 107 ist ein Ausatmungselement 108 angeordnet, welches ein Ventil umfasst oder als ein solches ausgebildet ist. Das Ausatmungselement 108 ist insbesondere dazu vorgesehen, während der Ausatmung des Nutzers ein Rückatmen in das Beatmungsgerät 1 zu verhindern.

Die Überwachungseinrichtung 21 ist hier mit einer nicht näher dargestellten Sensoreinrichtung wirkverbunden, welche einen oder mehrere Sensoren zur Erfassung von Geräteparametern und/oder Patientenparametern und/oder anderer für die Beatmung charakteristischer Größen aufweist.

Zum Beispiel umfasst die Überwachungseinrichtung 21 einen hier nicht näher gezeigten Drucksensor, welcher die Druckverhältnisse bezüglich der Atemschnittstelle 102 erfasst. Dazu ist der Drucksensor über einen Druckmessschlauch 110 mit der Atemschnittstelle 102 verbunden. Über einen Eingangsstutzen 111 ist der Druckmessschlauch 110 an die Überwachungseinrichtung 21 angebunden.

Des Weiteren dient die Überwachungseinrichtung 21 hier zur Ansteuerung der Beatmungseinrichtung 100. Die Überwachungseinrichtung 21 stellt einen notwendigen Minimaldruck bereit und kompensiert Druckschwankungen, die durch die Atemtätigkeit des Nutzers bedingt sind. Beispielsweise erfasst die Überwachungseinrichtung 21 auch den gegenwärtigen Druck in der Atemmaske 105 und regelt die Leistung der Beatmungseinrichtung 100-entsprechend nach, bis ein gewünschter Beatmungsdruck anliegt. Die zur Einstellung der Beatmungseinrichtung 100 benötigten Geräteparameter sowie die Gerätekonfiguration und/oder Gerätesoftware sind in einer Speichereinrichtung 31 hinterlegt. Die Überwachungseinrichtung 21 kann hier auch zur Erfassung von Patientenparametern ausgebildet sein. Die Überwachungseinrichtung 21 kann dazu mit Sensoren zur Messung der Atemexkursion, zur Messung einer Sauerstoffsättigung des Blutes und/oder zur Messung einer EEG-, einer EMG-, einer EOG- oder einer EKG-Aktivität ausgestattet sein.

Beispielsweise erfolgt durch die Überwachungseinrichtung 21 eine Regelung auf Soll-Geräteparameter, welche zuvor anhand der charakteristischen Atmung eines Nutzers individuell berechnet und festgelegt worden sind.

Es ist auch möglich, das die Beatmungseinrichtung 100 dynamisch und insbesondere je nach Atemphase des Nutzers angepasst wird. Beispielsweise kann anhand der Überwachungseinrichtung 21 ein Atemphasenwechsel erkannt werden, sodass je nach Atemphase ein höherer bzw. niedrigerer Druck bereitgestellt werden kann. Beispielsweise kann das Beatmungsgerät 1 als ein CPAP- oder APAP-Gerät ausgebildet sein. Das Beatmungsgerät 1 kann auch als ein Bilevel-Gerät ausgebildet sein. Beispielsweise reagiert das Beatmungsgerät 1 auf bestimmte Atemereignisse, wie beispielsweise Schnarchen, Atemabflachungen und/oder obstruktive Druckspitzen mit entsprechenden Einstellungen der Geräteparameter.

Die von der Überwachungseinrichtung 21 erfassten Druckverhältnisse werden zusammen mit weiteren Geräteparametern in einer Speichereinrichtung 31 hinterlegt. Zudem werden die von der Überwachungseinrichtung 21 eingestellten Druckverhältnisse bzw. die vorgenommenen Druckanpassungen ebenfalls als Geräteparameter in der Speichereinrichtung 31 hinterlegt. Auch die erfassten Patientenparameter können in der der Speichereinrichtung 31 hinterlegt werden. Zudem können Daten über Compliance und Maskendichtigkeit gespeichert werden.

Als Geräteparameter können beispielsweise ein Start-Therapie-Druck, ein maximaler Therapie-Druck, ein minimaler Therapie-Druck und/oder ein Zielvolumen und/oder andere zur Einstellung der Beatmungseinrichtung 100 geeignete Geräteparameter hinterlegt sein. Diese Geräteparameter werden von der Überwachungseinrichtung 21 zur Einstellung der Beatmungseinrichtung 100 aus der Speichereinrichtung 31 abgerufen.

Des Weiteren werden die über den Therapiezeitraum erfassten

Druckverhältnisse und/oder sonstigen Geräteparameter und/oder Patientenparameter im Rahmen von Therapieverläufen in der Speichereinrichtung 31 hinterlegt. Als Therapieverläufe können beispielsweise ein Flow-Verlauf, ein Druck-Verlauf und/oder ein Event-Verlauf registriert werden. Die Therapieverläufe werden der Speichereinrichtung 31 von der Überwachungseinrichtung 21 bereitgestellt, die diese Daten während der Therapie erfasst. Die in der Speichereinrichtung 31 hinterlegten Geräteparameter und/oder Patientenparameter und/oder Therapieverläufe werden abgerufen und zu einer oder mehrerer Therapiestatistiken ausgewertet. Die Therapiestatistik wird in der Speichereinrichtung 31 hinterlegt. Dabei können beispielsweise ein mittlerer Druck und/oder die Therapiedauer und/oder ein Leckageparameter ermittelt und abgespeichert werden.

Um die Therapiedaten einer therapeutischen oder diagnostischen Analyse unterziehen zu können oder um die Funktion des Beatmungsgerätes 1 überwachen und/oder einstellen zu können, ist eine Übertragung der Therapiedaten oder von Einstellungen oder von einer Geräte-ID an wenigstens einen Server 3 mittels einer Datenverarbeitungseinrichtung 2 vorgesehen. Die Datenverarbeitungseinrichtung 2 stellt hier die Komponenten bzw. Software bereit, damit der Server 3 die Daten des Beatmungsgerätes 1 interpretieren kann und umgekehrt.

Das Beatmungssystem 10 kann auch zwei oder mehr Server 3 umfassen, welche mit einem oder auch mehreren Beatmungsgeräten 1 verbunden sind.

Die Übertragung erfolgt mittels einer Übertragungseinrichtung 51 drahtlos und/oder drahtgebunden. Die Übertragungseinrichtung 51 kann ein Teil des Beatmungsgerätes sein oder als separates Bauteil dem Beatmungsgerät zugeordnet sein. Das Beatmungsgerät kann mit einem Modem 12, als Übertragungseinrichtung 51, verbunden sein, oder ein Modem 12 aufweisen.

Die Übertragung kann über eine oder mehrere Kabel-Schnittstellen erfolgen, beispielsweise USB, seriell, LAN, Datenbus etc. Die Übertragung kann auch über eine oder mehrere drahtlose Schnittstellen erfolgen, beispielsweise Mobilfunk, LPWAN, Bluetooth, Infrarot, Sigfox, Lora etc.

Der Server 3 kann wenigstens einen Server 13 und/oder wenigstens einen Personal Computer (PC) 23 umfassen oder auch als eine Rechnerwolke bzw. Cloud ausgebildet sein. So kann beispielsweise von einem PC 23 aus auf einen Webserver 13 zugegriffen werden, welcher wiederum über eine Netzwerkverbindung mit einem oder mehreren Beatmungsgeräten 1 verbunden ist.

Zudem können die Therapiedaten auch wenigstens teilweise auf einem transportablen Speichermedium 41 abgelegt werden. Das Speichermedium 41 ist beispielsweise als eine Speicherkarte oder eine Festplatte oder ein USB-Massenspeicher ausgebildet. Das Speichermedium 41 kann aus dem Gerät entnommen und über ein Lesegerät und beispielsweise einen Computer, Tablet-Computer, Smartphone ausgelesen werden.

Die Therapiedaten oder andere Daten können auch über ein im Gerät 1 befindliches und/oder an das Gerät 1 angeschlossenes Display 11 oder eine Nutzerschnittstelle 61 ausgelesen werden.

Über dieselben Schnittstellen kann das Gerät 1 mit neuen Konfigurationsdaten oder neuem Programmcode ausgestattet oder Funktionen im Gerät 1 aktiviert werden. Ein oder mehrere Datenspeicher 31, 41 im Gerät 1 werden also bevorzugt von außen beschrieben und/oder gelesen.

Von dem Server 3 aus kann ein Zugriff auf die Therapiedaten von einem entfernten Ort erfolgen, sodass eine ortsunabhängige Auswertung möglich ist. So können Betreuer (Ärzte, Provider...) auf die Daten im Beatmungssystem 10 zugreifen. Der Zugriff wird aus Datenschutz und Datensicherheitsgründen beispielsweise auf die selbst eingesetzten Geräte 1 beschränkt. Der Patient (Nutzer) bekommt beispielsweise nur auf sein Gerät 1 Zugriff. Fachpersonal wie Provider und Ärzte (Betreuer) bekommen beispielsweise auf die Geräte 1 aller ihrer Patienten Zugriff.

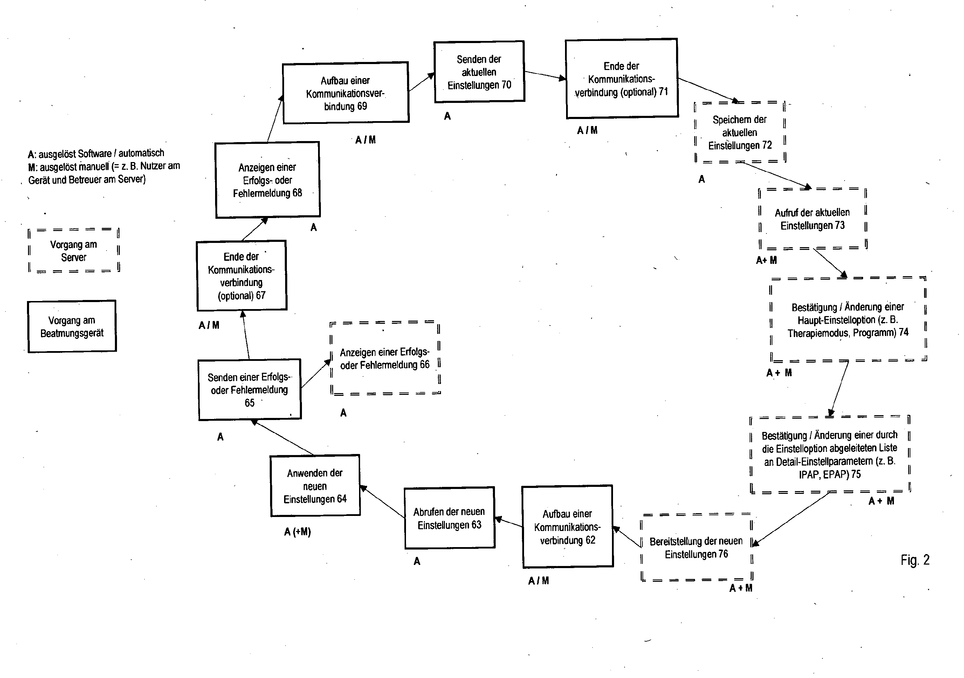

Figur 2 zeigt beispielhaft einen Kommunikationsablauf zwischen dem Beatmungsgerät 1 und dem Server 3.

Der Aufbau einer Kommunikationsverbindung zwischen einem Server und dem Beatmungsgerät 69 wird beispielsweise vom Beatmungsgerät (oder dem Server 3) automatisch oder manuell durch einen Anwender (Nutzer oder Betreuer) initiiert. Automatisch wird die Verbindung beispielsweise in einem voreingestellten oder einstellbaren Zeitintervall, beispielsweise alle 5 oder 60 Minuten, aufgebaut. Sobald die Kommunikationsverbindung etabliert ist erfolgt automatisch ein Senden der aktuellen Einstellungen des Beatmungsgerätes, die beispielsweise zumindest den Gerätetyp und die Firmware-Version und/oder eine Geräte-ID umfassen 70, an den Server 3. Optional kann nach dem Senden der aktuellen Einstellungen 70 die Kommunikationsverbindung zwischen Beatmungsgerät und Server 3 enden 71. Dieser Vorgang kann automatisch oder durch einen Anwender initiiert sein.

Automatisch erfolgt eine Speicherung der aktuellen Einstellungen auf dem Server 72.

Automatisch und/oder manuell durch einen Anwender initiiert erfolgt dann ein Aufrufen der aktuellen Einstellungen auf dem Server 73.

Automatisch und/oder manuell durch einen Anwender initiiert erfolgt dann eine Auswahl der gerätespezifischen Einstelloptionen, aus einer Datenbank des Servers, und ein Bereithalten der gerätespezifischen Einstelloptionen 74. Optional können diese auch bestätigt oder geändert werden.

Automatisch und/oder manuell durch einen Anwender initiiert erfolgt dann eine Auswahl der gerätespezifischen Einstellparameter für die Einstelloptionen 75.

Automatisch und/oder manuell durch einen Anwender initiiert erfolgt dann eine Bereitstellung der neuen Einstellung für das Beatmungsgerät 76.

Automatisch oder manuell durch einen Anwender initiiert erfolgt dann der Aufbau einer Kommunikationsverbindung zwischen dem Server und dem Beatmungsgerät 62.

Automatisch erfolgt dann das Abrufen der neuen Einstellungen 63 durch das Beatmungsgerät.

Automatisch, und/oder optional manuell durch einen Anwender initiiert, erfolgt dann das Anwenden der neuen Einstellungen 64. Automatisch erfolgt dann das Senden einer Erfolgs- oder Fehlermeldung 65.

Automatisch erfolgt das Anzeigen einer Erfolgs- oder Fehlermeldung 68. Diese kann optional oder ergänzend am Beatmungsgerät und/oder am Server angezeigt (66, 68) werden.

Die Erfolgs- oder Fehlermeldung kann auch als Benachrichtigung wie beispielsweise in Form einer Email, SMS, Whatsapp-Nachricht an ein Kommunikationsgerät des Nutzers und/oder Betreuers versendet werden.

Die Einstellungen, die der Server an das Beatmungsgerät sendet, können neben den Beatmungseinstellungen auch eine Auswahl von Text-/Sprach-Bausteinen sein, die das Beatmungsgerät oder mindestens eine damit verbundene Mensch-Maschine-Schnittstelle an seinen Nutzer ausgeben soll, insbesondere Meldungen oder Fragen. Die Fragen beziehen sich bevorzugt auf die Symptome der Erkrankung, die Nebenwirkungen der Beatmungstherapie, die Lebensqualität oder den Gesundheitszustand oder Fehlerzustände des Beatmungsgerätes. Die Text-/Sprachbausteine können im Gerät hinterlegt und durch die Ferneinstellung aktiviert werden, oder sie werden durch die Ferneinstellung selbst an das Beatmungsgerät geschickt.

Die Einstellungen sind üblicherweise Beatmungseinstellungen oder auch jede Anweisung, die eine Statusänderung am Gerät veranlasst wie beispielsweise Komforteinstellungen, Geräteeinstellungen (Displayhelligkeit, ...), Alarmeinstellungen, Dateneinstellungen oder auch Aktionen. Die Einstelloptionen sind dann beispielsweise Beatmungsdrücke, Beatmungsfrequenz, Volumen oder auch inspiratorischer Druck, exspiratorischer Druck, Backup-Frequenz, eine Textnachricht für das Gerätedisplay, Gerät neu booten, akustisches Signal geben, Beatmungsprogramm umschalten, Firmware aktualisieren.

Die dazugehörigen Einstellpärameter 75 sind dann beispielsweise die Druckwerte oder konkrete Frequenzen oder andere konkrete Werte oder frei eingebbare Texte oder Zeichen (für die Kommunikation).

Als Einstellungen gelten neben den dauerhaft wirksamen Einstellungen wie Firmware-Code, Beatmungsmodus, Beatmungsdrücke, Beatmungsfrequenz, Komfortparameter auch Aktionen, die per Fern-Einstellungen am Gerät mindestens einmalig ausgelöst werden, beispielsweise
- ein Neustart des Gerätes
- Senden bestimmter Daten aus dem Gerätespeicher
- Löschen bestimmter Daten im Gerätespeicher
- Senden einer Authentifizierung
- Durchführung mindestens eines Selbst-Tests des Gerätes
- Durchführung mindestens einer Aktion zur Reinigung des Gerätes oder von Zubehörteilen
- Durchführung mindestens eines Beatmungs-Manövers, beispieslweise Start oder Ende der Beatmung, Applikation eines vom normalerweise eingestellten Druck abweichenden Wert für einen begrenzten Zeitraum
- Darstellung einer bestimmten Information grafisch, optisch oder akustisch im Gerät

Ein erfindungsgemäß ebenfalls vorgesehener Anwendungsfall der Ferneinstellung ist ein Update des Beatmungsgerätes mit neuer Software oder Firmware. Der Server kann solche Updates automatisch oder durch den Betreuer initiiert an das Beatmungsgerät senden. Am Server können unterschiedliche Betreuer unterschiedliche Rechte zur Änderung von Geräteeinstellungen besitzen. Erfindnungsgemäß ist eine Benutzerrechte-Verwaltung des Servers, zur Vorgabe/Einstellung Benutzerrechte, vorgesehen. So könnte beispielsweise der Nutzer des Beatmungsgeräts selbst auch ein Betreuer des Servers sein und/oder nur eine eingeschränkte Menge an Einstellungen vornehmen dürfen (beispielsweise Komfortparameter) . Oder ein bestimmter (technischer) Betreuer darf ebenfalls nur eine eingeschränkte Anzahl Einstellungen ändern. Dies wird von der Nutzerrechte-Verwaltung des Servers gesteuert . oder ist durch diese vorgebbar.

Auf dem beschriebenen Weg der Ferneinstellung können auch Zubehör-Komponenten ferneingestellt werden, die mit dem Beatmungsgerät verbunden sind. Insbesondere Befeuchter, Schlauchheizung, Sensormodule (SpO2, FiO2, CO2), Alarmmodule, Akku- / Batterie-Module, Sauerstoffkonzentratoren, Sauerstoff-/Gas-Mischer. Abhängig von der gewählten Einstellung mindestens eines (Haupt-)Parameters kann es sein, dass der Server dem Betreuer dynamisch mehr oder weniger zusätzliche Neben-Einstellparameter anbietet oder vorschlägt. Beispielsweise werden bei Auswahl einer DruckAutomatik (APAP, auto-EPAP, Zielvolumen) eine untere und obere Druckgrenze voreingestellt oder vorgeschlagen. Beispielsweise werden bei Abschalten der Druckautomatik ein fester Druckwert voreingestellt oder vorgeschlagen oder untere und obere Druckgrenze. Beispielsweise werden bei Auswahl eines Zielvolumens eine untere und obere Druckgrenze voreingestellt oder vorgeschlagen. Beispielsweise werden bei Auswahl einer Frequenz eine untere und obere Druckgrenze voreingestellt oder vorgeschlagen. Die zugehörige Einstell-Logik des Beatmungsgerätes ist dafür beispielsweise auf dem Server derart gespiegelt abgebildet, dass diese Anpassungen der zur Verfügung gestellten Neben-Parameter erfolgt, ohne dass der Server nach Wahl des Haupt-Einstellparameters mit dem Beatmungsgerät kommunizieren und von diesem die Liste der Neben-Einstellparameter abfragen muss. Abhängig von der gewählten Einstellung mindestens einer Einstelloption kann es sein, dass der Server dem Betreuer dynamisch mehr oder weniger zusätzliche "sinnvolle" Einstelloptionen anbietet oder vorschlägt. Beispielsweise werden bei Abschalten der Druckautomatik eine Sicherheitsfrequenz voreingestellt oder vorgeschlagen. Beispielsweise werden bei Auswahl einer Frequenz eine untere und obere Druckgrenze voreingestellt oder vorgeschlagen. Die zugehörige Einstell-Logik des Beatmungsgerätes ist dafür beispielsweise auf dem Server derart gespiegelt abgebildet, dass diese Anpassungen der zur Verfügung gestellten Neben-Parameter erfolgt, ohne dass der Server nach Wahl des Haupt-Einstellparameters mit dem Beatmungsgerät kommunizieren und von diesem die Liste der Neben-Einstellparameter abfragen muss.

Das Verfahren sieht optional oder ergänzend vor, dass nach dem Bereitstellen / Senden einer Erfolgs- oder Fehlermeldung 65, die Kommunikationsverbindung zwischen Beatmungsgerät und Server 3 endet 67. Die Beendigung 67 der Kommunikationsverbindung kann automatisch, oder optional manuell durch einen Anwender initiiert, erfolgen.

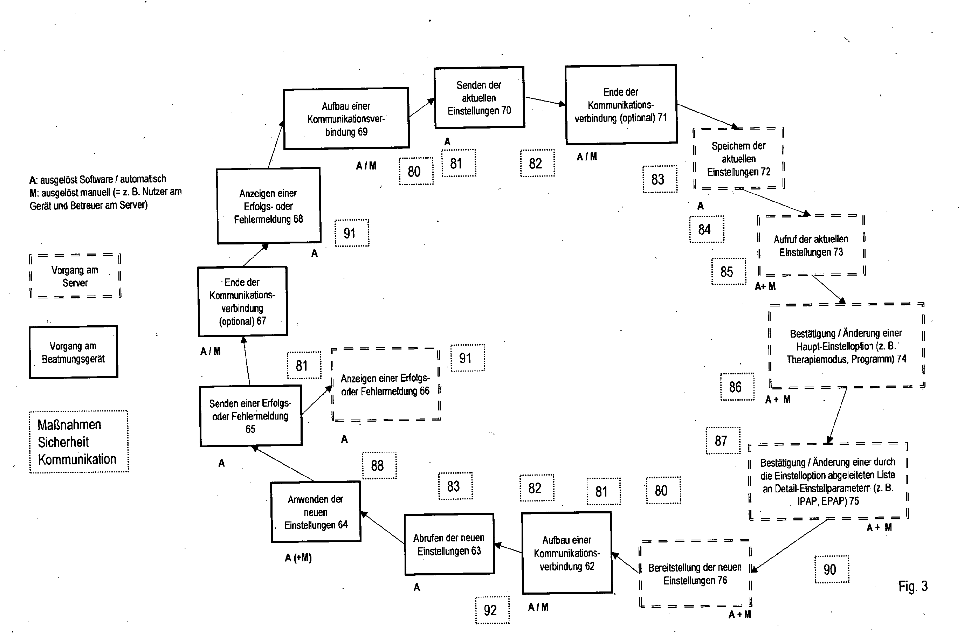

Figur 3 zeigt beispielhaft die Sicherheitsmechanismen, die bei einem Kommunikationsablauf zwischen dem Beatmungsgerät 1 und dem Server 3 erfolgen.

Bei dem Aufbau einer Kommunikationsverbindung zwischen einem Server und dem Beatmungsgerät 69 erfolgt eine Authentifizierung von Gerät und Server 80. Die Authentifizierung kann beispielsweise dadurch erfolgen, dass eine Prüfung von Geräte-Typ und Seriennummer oder eine Geräte-ID erfolgt. Die Authentifizierung kann alternativ oder ergänzend dadurch erfolgen, dass eine Prüfung der SIM-Kartennummer (beispielsweise bei Verwendung einer SIM-Karte in einem Modem) erfolgt. Die Authentifizierung kann alternativ oder ergänzend dadurch erfolgen, dass eine Prüfung der IP-Adresse des Servers erfolgt. Die Authentifizierung kann alternativ oder ergänzend dadurch erfolgen, dass zwischen Beatmungsgerät und Server eine gegenseitige Überprüfung von Public und/oder Private Zertifikaten erfolgt. Die Authentifizierung kann alternativ oder ergänzend dadurch erfolgen, dass eine Prüfung eines Security-ID-Codes des Gerätes erfolgt. Die Authentifizierung kann alternativ oder ergänzend dadurch erfolgen, dass eine Kommunikation des Beatmungsgerätes nur mit einem definierten Zugangspunkt (APN) möglich ist. Die Authentifizierung kann alternativ oder ergänzend dadurch erfolgen, dass eine spezifische, definierte Frage-Antwort-Sequenz zwischen Gerät und Server durchlaufen werden muss. Die Authentifizierung kann alternativ oder ergänzend dadurch erfolgen, dass eine Überprüfung der Kombination von IDs / Seriennummern mehrerer Hardware-Bestandteile des Gerätes erfolgen muss.

Sobald eine Kommunikationsverbindung aufgebaut ist, erfolgt eine Sicherung der Kommunikationsverbindung 81. Die Sicherung der Kommunikationsverbindung 81 kann beispielsweise dadurch erfolgen, dass ein bestimmter, definierter Zugangspunkt (privater APN) verwendet wird. Die Sicherung der Kommunikationsverbindung 81 kann alternativ oder ergänzend dadurch erfolgen, dass eine Ende-zu-Ende-Verschlüsselung zwischen Beatmungsgerät und Server erfolgt. Die Sicherung der Kommunikations-verbindung 81 kann alternativ oder ergänzend dadurch erfolgen, dass ein VPN-Kanal (virtuelles privates und in sich geschlossenes Kommunikationsnetz) von dem definierten Zugangspunkt (APN) bis zum Server aufgebaut ist.

Die Kommunikationsverbindung 81 kann alternativ oder ergänzend dadurch erfolgen, dass eine Firewall etabliert ist. Die Kommunikationsverbindung 81 kann alternativ oder ergänzend dadurch erfolgen, dass nicht benötigte Kommunikations-Möglichkeiten und Ports blockiert werden.

Erfindungsgemäß ist auch vorgesehen, dass die Kommunikation über die Kommunikations-verbindung verschlüsselt 82 erfolgt. Die Verschlüsselung 82 kann beispielsweise dadurch erfolgen, dass ein TLS- oder SSL-Standard oder Varianten davon verwendet werden. Die Verschlüsselung 82 kann alternativ oder ergänzend dadurch erfolgen, dass eine direkte Verschlüsselung mit einem Schlüssel erfolgt, dessen Länge größer oder gleich 8 bit ist. Die Verschlüsselung 82 kann alternativ oder ergänzend dadurch erfolgen, dass HTTPS oder SFTP oder Varianten davon angewendet werden.

Bei der Kommunikation erfolgt auch eine Überprüfung der Integrität der Daten. Die Daten sollen nicht (aus Versehen) bei der Kommunikation verfälscht werden können, beispielsweise durch schlechte Datenqualität.

Eine Prüfung der Integrität 83 kann beispielsweise dadurch erfolgen, dass eine Ergänzung einer Checksumme zu der Nachricht erfolgt. Eine Prüfung der Integrität 83 kann alternativ oder ergänzend dadurch erfolgen, dass eine Verwendung von Übertragungsprotokollen mit einer Datenprüfung beispielsweise http, MQTT, TCP/IP, GSM, Bluetooth erfolgt. Eine Prüfung der Integrität 83 kann alternativ oder ergänzend dadurch erfolgen, dass eine Ergänzung eines Hash-Codes zu der Nachricht erfolgt, wobei der Hash-Code aus dem Inhalt der Nachricht und einem vorab definierten Sicherheitsschlüssel berechnet wird. Eine Prüfung der Integrität 83 kann alternativ oder ergänzend dadurch erfolgen, dass eine Ergänzung eines Hash-Codes zu der Nachricht erfolgt, wobei der Code aus dem Inhalt der Nachricht und einem vorab definierten Sicherheitsschlüssel und einer Zufallskomponente berechnet wird.

Bei der Kommunikation erfolgt auch eine Kennzeichnung 84 mit einem Zeitstempel und/oder einer Geräte-ID und FW-Version. Eine Kennzeichnung 84 kann beispielsweise dadurch erfolgen, dass eine Ergänzung mindestens eines Zeitstempels (beispielsweise für Aktivierung und Empfang) erfolgt, um auch bei asynchroner Datenverarbeitung die chronologische Reihenfolge der angewandten Einstellungen ermitteln und die neueste bekannte Einstellung identifizieren zu können. Eine Kennzeichnung 84 kann beispielsweise dadurch erfolgen, dass eine Kennzeichnung der gespeicherten Einstellungen mit Geräte-Typ und FW-Version erfolgt, damit der Server bei einer späteren Veränderung der Einstellungen (dem Benutzer) nur geeignete Einstellmöglichkeiten vorschlagen kann.

Erfindungsgemäß ist auch ein Zugriffsschutz für den Server 3 und eine Prüfung der Zugriffsrechte für jeden Betreuer vorgesehen 85. Der Zugriffsschutz für den Server 85 kann alternativ oder ergänzend dadurch erfolgen, dass
- ein physikalischer Schutz des Servers vor unbefugter Manipulation besteht
- eine verschlüsselte Datenverbindung zwischen dem Browser / Frontend und dem Server für authorisierte Betreuer besteht
- ein Zugriff auf den Server nur für authorisierte Betreuer, abhängig von ihren individuellen Nutzerrechten eingeräumt wird
- ein Schutz vor elektronischem Zugriff auf den Server beispielsweise durch eine Firewall oder einen Security-Container, oder eine-Datentrennung erfolgt
- ein Schutz vor elektronischem Zugriff über den Browser / das Frontend auf den Server durch Maßnahmen verhindert wird wie beipielsweise eine Rechte-Eskalation oder Cross-Site-Scripting oder OWASP Top 10 Bedrohungen
- eine starke Authentifizierung der Betreuer am Server erfolgt, beispielsweise mit 2-Faktor-Authentifizierung oder durch biometrische Überprüfung (Fingerabdruck, Iris-Scan, ...).

Erfindungsgemäß ist auch eine eindeutige Zuordnung 86 eines Nutzers zu einem Beatmungsgerät vorgesehen.

Die eindeutige Zuordnung 86 wird beispielsweise durch eine Zuordnung eines Gerätes über seine Seriennummer oder Geräte-ID zu einem Nutzer sichergestellt. Die eindeutige Zuordnung 86 wird alternativ oder ergänzend durch Bestätigungs-Vorgänge während der Zuordnung sichergestellt, beispielsweise durch Senden einer Kennung zwischen Gerät und Server oder einer manuellen Bestätigung am Gerät. Die eindeutige Zuordnung 86 wird alternativ oder ergänzend durch eine Anzeige von Nutzer-name oder -ID und Geräte-Seriennummer oder Geräte-ID während des gesamten Einstellvorgangs (für den Betreuer) veranschaulicht und somit überwacht. Die eindeutige Zuordnung 86 wird alternativ oder ergänzend durch eine Kommunikation/einen Aufruf (an den Betreuer) zur Überprüfung der Seriennummer des Gerätes bei jedem Einstellvorgang unterstützt, beispielsweise auch dann, wenn Einstellungen mit einer unabhängigen Quelle, beispielsweise einem ERP-System vorgenommen werden. Die eindeutige Zuordnung 86 wird alternativ oder ergänzend durch eine Kommunikation (einen Anruf) mit dem Geräte-Nutzer während des Einstellvorganges sichergestellt. Dies beinhaltet eine Abfrage der Bestätigungsmeldung. Die eindeutige Zuordnung 86 wird alternativ oder ergänzend durch eine Kommunikation mit dem Geräte-Nutzer sichergestellt, indem dieser aufgefordert wird eine manuelle Eingabe oder Ausgabe eines, beispielsweise telefonisch mitgeteilten, Bestätigungs-Codes am Gerät, zur Aktivierung der geänderten Einstellungen, durchzuführen.

Erfindungsgemäß ist auch vorgesehen, dass der Server für das spezifische Beatmungsgerät, aus einer Vielzahl an Einstelloptionen und Einstellparameter, eine spezifische Vorauswahl geeigneter Einstelloptionen 74 und dazu passender Einstellparameter 75 anbietet 87. Dies erleichtert die Vorgabe der Einstelloptionen und Einstellparameter und verhindert die Vorgabe unsinniger Kombinationen. Die Vorauswahl geeigneter Einstelloptionen und dazu passender Einstellparameter 87 wird dadurch erreicht, dass der Server das Beatmungsgerät anhand seiner Geräte-ID und/oder des Geräte-Typs und/oder der Seriennummer und/oder der Firmware-Version identifiziert. Aus einer Datenbank identifiziert der Server dann lediglich solche Einstelloptionen und Einstellparameter, die zu dem individuellen Beatmungsgerät kompatibel sind. Die Vorauswahl geeigneter Einstelloptionen und dazu passender Einstellparameter 87 wird alternativ oder ergänzend dadurch erreicht, dass nach Veränderung jedes Einstellwertes (Einstelloptionen und/oder Einstellparameter) oder zumindest vor der Bestätigung der vollständigen neuen Einstellungen überprüft wird, ob alle Abhängigkeiten zwischen den Einstellwerten eingehalten wurden oder stimmig sind. Beispielsweise erfolgt eine Plausibilitätsprüfung von Obergrenze von Druckbereichen zu den Untergrenzen von Druckbereichen. Die Vorauswahl geeigneter Einstelloptionen und dazu passender Einstellparameter 87 wird alternativ oder ergänzend dadurch erreicht, dass eine automatische Anpassung von abhängigen Einstellwerten erfolgt. Die Vorauswahl geeigneter Einstelloptionen und dazu passender Einstellparameter 87 wird alternativ oder ergänzend dadurch erreicht, dass der Server das Beatmungsgerät anhand seiner Geräte-ID und/oder des Geräte-Typs und/oder der Seriennummer und/oder der Firmware-Version und auch den Nutzer des Gerätes (beispielsweise anhand einer Zuordnung Nutzer/Beatmungsgerät, die für den Server abrufbar ist) identifiziert und anhand hinterlegter Nutzerinformationen und hinterlegter Beatmungsgeräteinformationen automatisch nur solche Einstelloptionen und/oder Einstellparameter anbietet oder anwendet, die für die individuelle Kombination Nutzer/Beatmungsgerät geeignet sind. Dabei kann die Eignung für den Server abrufbar hinterlegt sein.

Erfindungsgemäß kann eine weitere Prüfung der Eignung 88 der vom Server bereitgestellten Einstellungen durch das Beatmungsgerät erfolgen. Diese Prüfung ist als zusätzliche Sicherheitsmaßnahme vorgesehen, da sich zwischen Senden der letzten Einstellungen und Abholen der neuen Einstellungen Änderungen ergeben haben können (beispielsweise die FW-Version sich geändert haben kann oder auch ein neues Beatmungsgerät demselben Patienten ausgehändigt worden sein kann).

Erfindungsgemäß kann eine weitere Prüfung der vom Server bereitgestellten Einstellungen durch den Nutzer erfolgen. Diese Prüfung ist als zusätzliche Sicherheitsmaßnahme vorgesehen, und sieht vor, dass der Nutzer die Übernahme der Einstellungen am Beatmungsgerät oder für das Beatmungsgerät bestätigt 89. Die Bestätigung der Übernahme 89 kann dadurch erfolgen, dass der Nutzer eine Bestätigungstaste am Gerät betätigt oder eine Übernahme akkustisch bestätigt. Die Bestätigung der Übernahme 89 kann auch dadurch erfolgen, dass der Nutzer dem Gerät einen Sicherheitscode eingeben muss oder dem Betreuer einen Code kommunizieren muss. Dieser Code kann dem Nutzer dauerhaft vorliegen oder für jede Änderung der Einstellungen jeweils neu kommuniziert werden beispielsweise in Form einer Email, SMS, Whatsapp-Nachricht an ein Kommunikationsgerät des Nutzers und/oder das Beatmungsgerät. Erfindungsgemäß kann eine Prüfung der Eignung der vom Server bereitgestellten Einstellungen dadurch erfolgen, dass Identität des Nutzers verifiziert 90 werden muss. Eine Prüfung der Identität 90 kann im Moment der Bereitstellung von neuen Einstellungen erfolgen. Eine Prüfung der Identität 90 kann dadurch erfolgen, dass ein Passwort oder Einmal-Passwort oder Sicherheitscode kommuniziert oder eingegeben werden muss. Eine Prüfung der Identität 90 kann dadurch erfolgen, dass eine Biometrische Überprüfung (beispielsweise Fingerabdruck, Iris-Scan, Stimm-Überprüfung) erfolgt.

Erfindungsgemäß kann eine Bestätigung/Quittierung 91 der vom Server bereitgestellten Einstellungen durch den Nutzer erfolgen. Die Quittierung 91 durch Nutzer dient der Sicherstellung der Wahrnehmung einer Meldung über die geplanten oder durchgeführten Änderungen und schaltet diese bevorzugt erst dann frei. Die Quittierung 91 durch Nutzer kann durch einen Mausclick oder Tastendruck oder eine Geste oder Touch-Eingabe oder eine Zustimmung per Sprache erfolgen.

Erfindungsgemäß kann eine Deaktivierungsmöglichkeit des Abrufens von Ferneinstellungen 92 vorgesehen sein. Dadurch kann zumindest bei Deaktivierung ein zusätzlicher Schutz vor Ferneinstellung des Beatmungsgerätes erfolgen. Die Deaktivierung 92 kann als Einstelloption direkt am Gerät getätigt werden. Die Deaktivierung kann nur das Senden von aktuellen Einstellungen oder das Senden / Abrufen von Einstellungen umfassen. Das Abrufen kann dabei nicht bei jedem Sendevorgang erfolgen, sondern nur bei bestimmten Vorgängen, beispielsweise durch manuelles oder zeitgesteuertes Auslösen.

1

## Patentansprüche

1. Verfahren zur sicheren Kommunikation in einem Beatmungssystem (10) zur Ferneinstellung eines Beatmungsgerätes (1) durch einen Server (3) umfassend, dass
- eine Authentifizierung (80) von Beatmungsgerät (1) und Server (3) durchgeführt wird
- eine Kommunikationsverbindung (81) zwischen dem Beatmungsgerät (1) und dem Server (3) gesichert wird
- eine Verschlüsselung (82) der Kommunikation zwischen dem Beatmungsgerät (1) und dem Server (3) vorgenommen wird
- die Integrität (83) der übermittelten Daten/Einstellungen überprüft wird
- eine Kennzeichnung (84) der übermittelten Daten/Einstellungen mit einem Zeitstempel und/oder einer Geräte-ID und/oder einer Firmware-Version stattfindet
- ein Zugriff auf den Server und/oder eine Prüfung der Zugriffsrechte (85) für den Server (3) geschützt wird
- eine eindeutige Zuordnung (86) eines Nutzers zu einem Beatmungsgerät (1) durchgeführt wird
- eine Vorauswahl (87) geeigneter Einstelloptionen (74) und dazu passender Einstellparameter (75) für ein individuelles Beatmunqsqerät (1) durch den Server (3) bereitgestellt wird, und
- die Eiqnunq (88) der vom Server (3) bereitgestellten Einstelloptionen (74) und Einstellparameter (75) durch das Beatmungsgerät (1) geprüft wird,
**dadurch gekennzeichnet, dass** der Server (3) lediglich solche Einstelloptionen (74) und Einstellparameter (75) aus einer Datenbank identifiziert, die zu dem individuellen Beatmungsgerät (1) kompatibel sind.

2. Verfahren nach Anspruch (1), bei dem eine Prüfung/Bestätigung (89) der vom Server (3) bereitgestellten Einstellungen durch den Nutzer des Beatmungsgerätes erfolgt, indem der Nutzer die Übernahme der Einstellungen am Beatmungsgerät (1) oder für das Beatmungsgerät bestätigt (89).

3. Verfahren nach Anspruch (1) oder (2), bei dem eine Identität des Nutzers verifiziert (90) wird.

4. Verfahren nach zumindest einem der vorhergehenden Ansprüche **dadurch gekennzeichnet, dass** folgende Schritte umfasst sind
- Aufbau einer Kommunikationsverbindung zwischen einem Server und einem Beatmungsgerät (6t)
- Senden der aktuellen Einstellungen, die zumindest den Gerätetyp und die Firmware-Version und/oder eine Geräte-ID umfassen (70)
- Speichern der aktuellen Einstellungen auf dem Server (72)
- Aufrufen der aktuellen Einstellungen auf dem Server (73)
- Auswahl der gerätespezifischen Einstelloptionen, aus einer Datenbank des Servers, und Bereithalten der gerätespezifischen Einstelloptionen (74)
- Definition einer ersten Einstelloption (75)
- Bereitstellung der ersten Einstelloption für das Beatmungsgerät (76)
- Aufbau einer Kommunikationsverbindung zwischen einem Server und einem Beatmungsgerät (62)
- Abrufen der neuen Einstellungen (63)
- Anwenden der neuen Einstellungen (64)
- Bereitstellen / Senden einer Erfolgs- oder Fehlermeldung (65)
- Anzeigen einer Erfolgs- oder Fehlermeldung (68).

5. Verfahren nach zumindest einem der vorhergehenden Ansprüche, bei dem nach dem Anwenden der neuen Einstellungen (64) der ersten Einstelloption (75) sukzessiv zumindest eine zweite Einstelloption (75') definiert, bereitgestellt und angewendet wird.

6. Verfahren nach zumindest einem der vorhergehenden Ansprüche, bei dem die erste Einstelloption (75) und zumindest eine zweite Einstelloption (75') zumindest teilweise gleichzeitig an das Beatmungsgerät gesendet und angewendet werden.

7. Verfahren nach zumindest einem der vorhergehenden Ansprüche, bei dem nach dem Senden der aktuellen Einstellungen (70) die Kommunikationsverbindung zwischen Beatmungsgerät und Server (3) endet (71).

8. Verfahren nach zumindest einem der vorhergehenden Ansprüche, bei dem nach dem Bereitstellen / Senden einer Erfolgs- oder Fehlermeldung (65), diese am Beatmungsgerät und am Server angezeigt (66, 68) wird.

9. Verfahren nach zumindest einem der vorhergehenden Ansprüche, bei dem nach dem Bereitstellen / Senden einer Erfolgs- oder Fehlermeldung (65), die Kommunikationsverbindung zwischen Beatmungsgerät und Server (3) endet (67).

10. Verfahren nach zumindest einem der vorhergehenden Ansprüche mit den Schritten;
- Aufbau einer Kommunikationsverbindung zwischen einem Server und einem Beatmungsgerät
- Senden der aktuellen Einstellungen, die zumindest den Gerätetyp und die Firmware-Version und/oder eine Geräte-ID umfassen, von dem Beatmungsgerät an dem Server
- Identifikation des Gerätes durch den Server
- Auswahl der gerätespezifischen Einstelloptionen durch Server, aus einer Datenbank des Servers, und Bereithalten der gerätespezifischen Einstelloptionen
- Definition einer ersten Einstelloption durch den Server, sowie Übertragung der ersten Einstelloption durch den Server
- Überprüfung der ersten Einstelloption durch das Beatmungsgerät
- Bestätigung der ersten Einstelloption durch das Beatmungsgerät
- Definition, sowie Übertragung der Einstellparameter der ersten Einstelloption durch den Server
- Überprüfung der Einstellparameter der ersten Einstelloption durch das Beatmungsgerät
- Bestätigung der Einstellparameter der ersten Einstelloption von durch das Beatmungsgerät.

11. Verfahren nach zumindest einem der vorhergehenden Ansprüche, bei dem eine Überprüfung der ersten Einstelloption durch das Beatmungsgerät erfolgt und, wenn diese nicht für das Gerät nicht geeignet sind, senden einer Fehler-ID an den Server und abspeichern der Fehler-ID zusammen mit der Geräte-ID, insbesondere Gerätetyp und Firmware-Version, des Beatmungsgerätes.

12. Verfahren nach zumindest einem der vorhergehenden Ansprüche, bei dem nach der Definition einer ersten Einstelloption durch den Server, sowie der Übertragung der ersten Einstelloption durch den Server zumindest eine zweite Einstelloption definiert und übertragen wird.

13. Verfahren nach zumindest einem der vorhergehenden Ansprüche, bei dem eine Überprüfung der ersten Einstelloption durch das Beatmungsgerät erfolgt und, wenn diese für das Gerät geeignet sind, senden einer Bestätigungs-ID an den Server zusammen mit den übernommenen Werten der ersten Einstelloption und abspeichern der Bestätigungs-ID zusammen mit der Geräte-ID, insbesondere Gerätetyp und Firmware-Version, des Beatmungsgerätes.

14. Verfahren nach zumindest einem der vorhergehenden Ansprüche, bei dem eine Signalisierung der geänderten Einstellung an den Nutzer des Beatmungsgerätes auf dem Display des Beatmungsgerätes und/oder einem persönlichen Kommunikationsgerät des Benutzers.

15. Verfahren nach zumindest einem der vorhergehenden Ansprüche, bei dem ein Betreuer an dem Server einen Meldungstext für den Nutzer eingibt und dieser Meldungstext von dem Server an das Beatmungsgerät übermittelt wird und dort auf dem Display angezeigt wird oder von dem Server an ein persönlichen Kommunikationsgerät des Benutzers übermittelt wird.

16. Verfahren nach zumindest einem der vorhergehenden Ansprüche, bei dem ein Betreuer von dem Server auf dem Display eine Rückmeldung erhält, ob und wann die neuen Einstellungen vom Beatmungsgerät übernommen wurden oder ob es zu einem Fehler kam.

17. Verfahren nach zumindest einem der vorhergehenden Ansprüche, bei dem sich ein Betreuer vor dem Absenden neuer Einstellungen für das Beatmungsgerät an dem Server authentifizieren muss und sich ein Nutzer vor dem Empfangen neuer Einstellungen für das Beatmungsgerät authentifizieren muss.

## Claims

1. A method for secure communication in a breathing system (10) for remote setting of a ventilator (1) via a server (3), comprising that
- an authentication (80) of the ventilator (1) and the server (3) is performed
- a communication connection (81) between the ventilator (1) and the server (3) is ensured
- an encryption (82) of the communication between the ventilator (1) and the server (3) occurs
- the integrity (83) of the transmitted data/settings is checked
- a labeling (84) of the transmitted data/settings with a time stamp and/or a device ID and/or a firmware version takes place
- access to the server and/or a check of the access rights (85) for the server (3) is protected
- a unique assignment (86) of a user to a ventilator (1) is performed
- a preselection (87) of suitable setting options (74) and matching setting parameters (75) for an individual ventilator (1) is provided by the server (3), and
- the suitability (88) of the setting options (74) and setting parameters (75) provided by the server (3) is checked by the ventilator (1),
**characterized in that**
the server (3) identifies only those setting options (74) and setting parameters (75) from a database which are compatible with the individual ventilator (1).

2. The method according to claim (1), in which a check/confirmation (89) of the settings provided by the server (3) takes place by the user of the ventilator in that the user confirms (89) the adoption of the settings at the ventilator (1) or for the ventilator.

3. The method according to claim (1) or (2), in which an identity of the user is verified (90).

4. The method according to at least one of the preceding claims, **characterized in that** the following steps are comprised
- establishing a communication connection between a server and a ventilator (6t)
- sending the current settings which comprise at least the device type and the firmware version and/or a device ID (70)
- storing the current settings on the server (72)
- calling up the current settings on the server (73)
- selection of the device-specific setting options, from a database of the server, and keeping the device-specific setting options available (74)
- definition of a first setting option (75)
- providing the first setting option for the ventilator (76)
- establishing a communication connection between a server and a ventilator (62)
- calling up the new settings (63)
- applying the new settings (64)
- providing/sending a success or error message (65)
- displaying a success or error message (68).

5. The method according to at least one of the preceding claims, in which, after the application of the new settings (64) of the first setting option (75), at least one second setting option (75') is successively defined, provided and applied.

6. The method according to at least one of the preceding claims, in which the first setting option (75) and at least one second setting option (75') are sent at least partially simultaneously to the ventilator and applied.

7. The method according to at least one of the preceding claims, in which, after the sending of the current settings (70), the communication connection between the ventilator and the server (3) ends (71).

8. The method according to at least one of the preceding claims, in which, after the providing/sending of a success or error message (65), it is displayed on the ventilator and on the server (66, 68).

9. The method according to at least one of the preceding claims, in which, after the providing/sending of a success or error message (65), the communication connection between the ventilator and the server (3) ends (67).

10. The method according to at least one of the preceding claims with the steps;
- establishing a communication connection between a server and a ventilator
- sending the current settings, which comprise at least the device type and the firmware version and/or a device ID, from the ventilator to the server
- identification of the device by the server
- selection of the device-specific setting options by the server, from a database of the server, and keeping the device-specific setting options available
- definition of a first setting option by the server, as well as transmission of the first setting option by the server
- checking of the first setting option by the ventilator
- confirmation of the first setting option for the ventilator
- definition, as well as transmission of the setting parameters of the first setting option by the server
- checking of the setting parameters of the first setting option by the ventilator
- confirmation of the setting parameters of the first setting option by the ventilator.

11. The method according to at least one of the preceding claims, in which a check of the first setting option by the ventilator takes place and, when they are not suitable for the device, sending an error ID to the server and storing the error ID together with the device ID, in particular device type and firmware version, of the ventilator.

12. The method according to at least one of the preceding claims, in which, after the definition of a first setting option by the server, as well as the transmission of the first setting option by the server, at least one second setting option is defined and transmitted.

13. The method according to at least one of the preceding claims, in which a check of the first setting option by the ventilator takes place and, when they are suitable for the device, sending a confirmation ID to the server together with the adopted values of the first setting option and storing the confirmation ID together with the device ID, in particular device type and firmware version, of the ventilator.

14. The method according to at least one of the preceding claims, in which a signaling of the modified setting to the user of the ventilator on the display of the ventilator and/or a personal communication device of the user.

15. The method according to at least one of the preceding claims, in which a caregiver enters a message text at the server for the user and this message text is transmitted from the server to the ventilator and is shown there on the display or is transmitted from the server to a personal communication device of the user.

16. The method according to at least one of the preceding claims, in which a caregiver receives a response from the server on the display as to whether and when the new settings have been adopted by the ventilator or whether there was an error.

17. The method according to at least one of the preceding claims, in which a caregiver must be authenticated at the server before sending out new settings for the ventilator and a user must be authenticated before receiving new settings for the ventilator.

## Revendications

1. Procédé de communication sécurisée dans un système de respiration (10) pour le réglage à distance d'un appareil respiratoire (1) par un serveur (3), comportant
- l'exécution d'une authentification (80) de l'appareil respiratoire (1) et du serveur (3)
- la sécurisation d'une liaison de communication (81) entre l'appareil respiratoire (1) et le serveur (3)
- la réalisation d'un cryptage (82) de la communication entre l'appareil respiratoire (1) et le serveur (3)
- la vérification de l'intégrité (83) des données/réglages transmis
- la réalisation d'une identification (84) des données/réglages transmis à l'aide d'un horodatage et/ou d'un ID d'appareil et/ou d'une version de logiciel
- la protection d'un accès au serveur et/ou d'une vérification des droits d'accès (85) pour le serveur (3)
- l'exécution d'une association univoque (86) d'un utilisateur à un appareil respiratoire (1)
- la mise à disposition d'une présélection (87) d'options de réglage (74) appropriées et de paramètres de réglage (75) adaptés à celles-ci pour un appareil respiratoire (1) individuel par le serveur (3), et
- la vérification par l'appareil respiratoire (1) de l'adéquation (88) des options de réglage (74) et des paramètres de réglage (75) mis à disposition par le serveur (3),
**caractérisé en ce que**
le serveur (3) identifie seulement les options de réglage (74) et les paramètres de réglage (75) compatibles avec l'appareil respiratoire (1) individuel à partir d'une base de données.

2. Procédé selon la revendication (1), dans lequel une vérification/confirmation (89) des réglages mis à disposition par le serveur (3) est effectuée par l'utilisateur de l'appareil respiratoire, où l'utilisateur confirme (89) de l'acquisition des réglages sur l'appareil respiratoire (1) ou pour l'appareil respiratoire.

3. Procédé selon la revendication (1) ou (2), dans lequel une identité de l'utilisateur' est vérifiée (90).

4. Procédé selon l'une au moins des revendications précédentes, **caractérisé en ce que** celui-ci comporte les étapes suivantes
- établissement d'une liaison de communication entre un serveur et un appareil respiratoire (6t)
- envoi des réglages actuels, lesquels comportent au moins le type d'appareil et la version du logiciel et/ou un ID d'appareil (70)
- enregistrement des réglages actuels sur le serveur (72)
- consultation des réglages actuels sur le serveur (73)
- sélection des options de réglage spécifiques à l'appareil, à partir d'une base de données du serveur, et mise à disposition des options de réglage spécifiques à l'appareil (74)
- définition d'une première option de réglage (75)
- mise à disposition de la première option de réglage pour l'appareil respiratoire (76)
- établissement d'une liaison de communication entre un serveur et un appareil respiratoire (62)
- extraction des nouveaux réglages (63)
- application des nouveaux réglages (64)
- mise à disposition / envoi d'un message de réussite ou d'erreur (65)
- affichage d'un message de réussite ou d'erreur (68).

5. Procédé selon l'une au moins des revendications précédentes, dans lequel au moins une deuxième option de réglage (75') est définie, mise à disposition et appliquée successivement après l'application des nouveaux réglages (64) de la première option de réglage (75).

6. Procédé selon l'une au moins des revendications précédentes, dans lequel la première option de réglage (75) et au moins une deuxième option de réglage (75') sont envoyées à l'appareil respiratoire et appliquées au moins partiellement simultanément.

7. Procédé selon l'une au moins des revendications précédentes, dans lequel la liaison de communication entre l'appareil respiratoire et le serveur (3) se termine (71) après l'envoi des réglages actuels (70).

8. Procédé selon l'une au moins des revendications précédentes, dans lequel, après la mise à disposition / l'envoi d'un message de réussite ou d'erreur (65), celui-ci est affiché sur l'appareil respiratoire et sur le serveur (66, 68).

9. Procédé selon l'une au moins des revendications précédentes, dans lequel la liaison de communication entre l'appareil respiratoire et le serveur (3) se termine (67) après la mise à disposition / l'envoi d'un message de réussite ou d'erreur (65).

10. Procédé selon l'une au moins des revendications précédentes, comprenant les étapes suivantes ;
- établissement d'une liaison de communication entre un serveur et un appareil respiratoire
- envoi des réglages actuels, lesquels comportent au moins le type d'appareil et la version du logiciel et/ou un ID d'appareil, de l'appareil respiratoire au serveur
- identification de l'appareil par le serveur
- sélection des options de réglage spécifiques à l'appareil, à partir d'une base de données du serveur, et mise à disposition des options de réglage spécifiques à l'appareil
- définition d'une première option de réglage par le serveur, et transmission de la première option de réglage par le serveur
- vérification de la première option de réglage par l'appareil respiratoire
- confirmation de la première option de réglage par l'appareil respiratoire
- définition et transmission des paramètres de réglage de la première option de réglage par le serveur
- vérification des paramètres de réglage de la première option de réglage par l'appareil respiratoire
- confirmation des paramètres de réglage de la première option de réglage par l'appareil respiratoire.

11. Procédé selon l'une au moins des revendications précédentes, dans lequel une vérification de la première option de réglage est effectuée par l'appareil respiratoire et, lorsque celle-ci n'est pas appropriée pour l'appareil, un ID d'erreur est envoyé au serveur et l'ID d'erreur est enregistré conjointement avec l'ID d'appareil, en particulier le type d'appareil et la version de logiciel, de l'appareil respiratoire.

12. Procédé selon l'une au moins des revendications précédentes, dans lequel au moins une deuxième option de réglage est définie et transmise après la définition d'une première option de réglage par le serveur et la transmission de la première option de réglage par le serveur.

13. Procédé selon l'une au moins des revendications précédentes, dans lequel une vérification de la première option de réglage est effectuée par l'appareil respiratoire et, lorsque celle-ci est appropriée pour l'appareil, un confirmation d'ID est envoyée au serveur conjointement avec les valeurs récupérées de la première option de réglage et l'ID de confirmation est enregistrée conjointement avec l'ID d'appareil, en particulier le type d'appareil et la version de logiciel, de l'appareil respiratoire.

14. Procédé selon l'une .au moins des revendications précédentes, dans lequel le réglage modifié est indiqué à l'utilisateur de l'appareil respiratoire sur l'écran de l'appareil respiratoire et/ou sur un appareil de communication personnel de l'utilisateur.

15. Procédé selon l'une au moins des revendications précédentes, dans lequel un superviseur saisit un texte de message destiné à l'utilisateur sur le serveur et ledit texte de message est transmis du serveur à l'appareil respiratoire, où il est affiché sur l'écran ou transmis du serveur à un appareil de communication personnel de l'utilisateur.

16. Procédé selon l'une au moins des revendications précédentes, dans lequel un superviseur reçoit une réponse du serveur sur l'écran, indiquant si et quand les nouveaux réglages de l'appareil respiratoire ont été récupérés ou si une erreur a été rencontrée.

17. Procédé selon l'une au moins des revendications précédentes, dans lequel un superviseur doit s'authentifier sur le serveur avant l'envoi de nouveaux réglages pour l'appareil respiratoire et un utilisateur doit s'authentifier avant la réception de nouveaux réglages pour l'appareil respiratoire.
